(19) Europäisches Patentamt
European Patent Office
Office européen des brevets

(11) **EP 2 676 686 B1**

(12) **EUROPÄISCHE PATENTSCHRIFT**

(45) Veröffentlichungstag und Bekanntmachung des Hinweises auf die Patenterteilung:
**08.04.2015 Patentblatt 2015/15**

(51) Int Cl.:
*A61L 31/02* *(2006.01)* *C22C 19/07* *(2006.01)*

(21) Anmeldenummer: **13167767.6**

(22) Anmeldetag: **15.05.2013**

(54) **Stent aus einer Kobaltlegierung**

Stent made of a cobalt alloy

Stent en alliage de cobalt

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.06.2012 US 201261660820 P**

(43) Veröffentlichungstag der Anmeldung:
**25.12.2013 Patentblatt 2013/52**

(73) Patentinhaber: **Biotronik AG**
**8180 Bülach (CH)**

(72) Erfinder: **Gerold, Bodo**
**97753 Karlstadt (DE)**

(74) Vertreter: **Lindner-Vogt, Karin L.**
**Biotronik SE & Co. KG**
**Corporate Intellectual Properties**
**Woermannkehre 1**
**12359 Berlin (DE)**

(56) Entgegenhaltungen:
**EP-A1- 1 655 384 WO-A1-98/35067**
**WO-A1-2011/118615 WO-A1-2012/068358**
**US-A1- 2010 114 304**

## Beschreibung

Die Erfindung betrifft einen Stent, der ganz oder in Teilen aus einer Kobaltlegierung besteht.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch A-neurysmenstents bekannt, die vorrangig zur Abdichtung des Aneuryrismas dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Geweberveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare (hier als biokorrodierbar bezeichnete) Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder Ti-A16Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Die vorliegende Erfindung befasst sich mit nicht biodegradierbaren Implantatwerkstoffen, insbesondere Basislegierungen des Kobalts.

Stents müssen die Fähigkeit besitzen große plastische Dehnungen zu ertragen und ihren Größe bzw. Durchmesser zu behalten, wenn sie aufgeweitet sind. Grundsätzlich gilt, der ideale Stent sollte:

- ◦ ein geringes Profil besitzen; Dies umschließt die Tauglichkeit auf einen Ballonkatheter gecrimped zu werden.
- ◦ ein gutes Aufweitverhalten zeigen; Wenn der Stent in die Läsion eingeführt und der Ballon aufgeblasen wird, soll sich der Stent gleichmäßig aufweiten, um sich der Gefäßwand anzupassen.
- ◦ eine ausreichende Radialfestigkeit und einen vernachlässigbaren Recoil besitzen; Wenn der Stent einmal gesetzt ist, soll er den Rückstellkräften der atherosklerotischen Gefäßwand widerstehen und nicht kollabieren.
- ◦ eine ausreichende Flexibilität gegenüber Biegung besitzen; So kann der Stent auch durch Gefäße und Stenosen mit geringen Durchmesser befördert werden.
- ◦ eine angemessene Röntgensichtbarkeit bzw. MRI-Kompatibilität besitzen; So kann der Mediziner die Implantation und Lage des Stents in vivo beurteilen.
- ◦ geringe Thrombogenität besitzen; Das Material sollte biokompatibel sein und insbesondere die Anlagerung und Verklumpung von Blutplättchen vermeiden.
- ◦ die Möglichkeit zur Wirkstofffreisetzung haben; Dies dient insbesondere der Restinosevermeidung.

Die Anforderungen sprechen insbesondere die mechanischen Eigenschaften des Materials an, aus dem der Stent gefertigt ist. Die klassische Materialien 316L, MP53N und L-605, die für den Bau ballonexpandierbarer Stents verwendet werden, haben mechanische Nachteile, die die Freiheit bei der Stent-Design-Entwicklung und im Einsatz einengen:

(i) zu geringe (Zug-)Festigkeit Rm und plastische Dehnung At In der Folge sind der Kollapsdruck bzw. die Radialfestigkeit geringer, so dass dickere Stent-Streben notwendig sind, was zu einem größeren Lumenverlust bei der Implantation führt, die Einheilung (Endothelialisierung) in die Gefäßwand verzögert und die Freiheiten bei der geometrischen Stent-Design-Entwicklung eingeschränkt. Durch dickere Streben wird der Stent zudem steifer, was die Kurvengängigkeit herabsetzt

(ii) zu hohe Streckgrenze Rp0.2 (engl. YTS: yield tensile strength) Es resultiert eine starke elastische Rückfederung, was die Crimpbarkeit verschlechtert, zu einem dickeren Crimpprofil führt und einen höheren Recoil (Lumenverlust nach Aufweitung) bedingt.

Weiterhin muss die Biokompatibilität des Materials sichergestellt sein. Dabei wird immer wieder Nickel als Verursacher von Allergien oder lokalen sowie systemischen Unverträglichkeiten angeführt. Es besteht dem entsprechend ein Bedarf an nickelfreien Werkstoffen für den medizinischen Einsatz.

DE 197 04 530 A1 beschreibt eine nickelfreie, austenitische Kobalt-Basislegierung zur Vermeidung von Allergien bei verschiedenen Gebrauchsgegenständen, darunter auch Implantate, mit hoher Korrosionsbeständigkeit und guter Umformbarkeit. Nickel als Stabilisator für den austenitischen Zustand wird hier durch Zugabe von Titan und/oder Niob (zusammen 4 - 6 Gew.%) ersetzt. Die Legierung enthält zudem Cr (10 - 18 Gew.%), Fe (5 - 20 Gew.%) sowie Mo und W (zusammen 4 - 8 Gew.%, wobei der Gehalt an W halb so groß ist, wie der von Mo). Weiterhin kann die Legierung Cu (0 - 2 Gew.%), Mn (0 - 3 Gew.%), A1 (0 - 3 Gew.%), Si (0 - 1 Gew.%), C (0 - 0.1 Gew.%) und N (0 - 0.1 Gew.%) enthalten. Nachteile dieser Legierung ist eine zu geringe Duktilität und speziell eine zu gering Röntgensichtbarkeit für den Einsatz als Stent-Werkstoff. Zudem gelten Cu und Al nicht als biokompatibel.

US 3,865,585 beschreibt eine nickelfreie Kobalt-Basislegierungen mit Cr (26 - 31 Gew.%), Mo (4 - 6,5 Gew.%), Si (0 - 2 Gew.%), Fe (0 - 1 Gew.%), B (0 - 0,5 Gew.%), C (0 - 0,5 Gew.%), N (0.15 - 0.5 Gew.%), wobei der kumulierte Gehalt an C und N nicht höher als 0,7 Gew.% beträgt. Die Duktilität der Legierung ist mit weniger als 20% jedoch sehr gering und nicht für Stents geeignet.

DE 36 24 377 A1 schlägt eine Kobalt-Basislegierung mit folgender Zusammensetzung für medizinische Implantate und festsitzende Dentalprothesen vor: Cr (15 - 24 Gew.%), Fe (2 - 15 Gew.%), Mo (3 - 10 Gew.%), N (0 - 0.05 Gew.%) und C (0 - 0.05 Gew.%). Die Duktilität ist mit rund 10% sehr gering und nicht für Stents geeignet.

In WO2011/118615 (EP2551363) werden nickelfreie, Chrom, Wolfram und Eisen enthaltende Kobaltlegierungen für Stents beschrieben.

In US2010114304 werden nickelfreie oder nickelarme, Kobalt, Chrom, Eisen, Mangan, Kohlenstoff, Stickstoff, Kupfer sowie ein Platingruppenmetall oder Gold enthaltende Legierungen für Stents beschirieben.

Demnach besteht anhaltender Bedarf an einem nickelfreien metallischen Implantatwerkstoff mit hinreichend hoher Duktilität, der zur Herstellung von Stents geeignet ist.

Der erfindungsgemäße Stent löst oder mindert ein oder mehrere der zuvor geschilderten Probleme. Der Stent besteht ganz oder in Teilen aus einer Kobaltlegierung der Zusammensetzung:

| Co: | 18.36 - 66.85 Gew.% |
|-----|---------------------|
| Cr: | 17.0 - 30.0 Gew.% |
| Mn: | 4.0 - 10.0 Gew.% |
| W: | 9.0 - 18.0 Gew.% |
| Fe: | 3.0 - 20.0 Gew.% |
| C: | 0.03 - 0.5 Gew.% |
| N: | 0.1 - 1.0 Gew.% |
| Si: | 0 - 2.0 Gew.% |
| O: | 0 - 0.05 Gew.% |

wobei sich die genannten Legierungskomponenten und herstellungsbedingte Verunreinigungen auf 100 Gew.% summieren und

(i) eine Wertzahl PRE für Korrosionsbeständigkeit, die sich aus den Gew.%-Anteilen der genannten Legierungskomponenten nach der Formel (1)

$$PRE = [Cr] + 1.65 \times [W] + 30 \times [N] \tag{1}$$

ergibt, im Bereich von 34 - 89, bevorzugt 46 - 73, besonders bevorzugt 46 - 58 liegt; und

(ii) für den Gehalt an Stickstoff und Kohlenstoff folgende Einschränkungen nach den Formeln (2) und (3) gelten

$$0,15 \leq C + N \leq 1,00 \qquad (2)$$

$$0,25 \leq C/N \leq 1,00 \qquad (3).$$

Die erfindungsgemäß verwendete Co-Basislegierungen ist korrosionsbeständig, reibverschleißbeständig und besitzt eine hohe Festigkeit, die durch geeignete Wärmebehandlungen noch weiter erhöht werden kann, und gleichzeitig eine hohe Duktilität sowie ausgezeichneter Röntgensichtbarkeit. Die weiteren Legierungsbestandteile stabilisieren den Austenit, so dass die Legierung vorzugsweise vollständig in austenitischer Modifikation vorliegt.

Die erfindungsgemäß verwendeten Legierungen besitzen eine sehr hohe Festigkeit Rm von > 1000 MPa, bevorzugt > 1100 MPa. Die hohe Festigkeit ermöglicht es im Stent-Design dünne Strukturen zu realisieren, die dem Stent dennoch eine hohe Radialfestigkeit von > 1.5 bar (150 kPa) verleihen.

Die erfindungsgemäß verwendeten Legierungen besitzen ferner ein sehr ausgeprägtes Kaltverfestigungsverhalten ausgedrückt im Streckgrenzenverhältnis Rp/Rm (engl. YTS/UTS), das besonders klein sein sollte. Rp/Rm sollte < 0.75, bevorzugt < 0.60, besonders bevorzugt < 0.55 sein. Diese Eigenschaft ist insbesondere bedeutsam für die Steuerung der Verformung beim Crimpen und bei der Stentaufweitung. Es wird ein homogenes Öffnungsverhalten angestrebt, das von den mechanischen Eigenschaften des Materials aber auch dem Stent-Design abhängt, welches wiederum mit mehr Freiheiten entwickelt werden kann, wenn die mechanischen Eigenschaften des Materials es zulassen.

Die erfindungsgemäßen Legierungen zeigen weiterhin eine ausgezeichnete Umformbarkeit bei Raumtemperatur. Der Verformungsgrad (Bruchdehnung) At liegt bei > 40% bevorzugt > 50%, insbesondere > 60%.

Die erfindungsgemäßen Legierungen können durch geeignete, insbesondere mehrstufige Wärmebehandlungen zusätzlich gehärtet werden.

Im Ergebnis werden austenitische CoCrMnWFe-Legierungen bereitgestellt, die frei von Ni sind. Aus diesem Material gefertigte Stents weisen eine höhere Radialfestigkeit, ein homogenes Öffnungsverhalten, größere Dilatationsreserven (Nominaldurchmesser + 0.5 mm) und eine bessere Crimpbarkeit (geringerer Durchmesser) bei gleichzeitig verringertem Stegquerschnitt (besseres Einheilverhalten) gegenüber herkömmlichen Stents aus. Auf Grund der Nickel-Freiheit resultiert eine stark verbesserte Biokompatibilität. Zudem ist die Beständigkeit gegen Lochfraß, gegen Abrieb und gegen Reibkorrosion insbesondere in den Situationen verbessert, in denen zwei oder mehrere Stents sich gegenseitig überlappen.

Die Elemente C, N und Mn dienen ferner als Nickelersatz zur Stabilisierung des austenitischen (kubisch flächenzentriert) Zustands.

Ein Anteil von Mn an der Legierung beträgt vorzugsweise 6.0 bis 10.0 Gew.%, insbesondere 7.0 bis 9.0 Gew.% beträgt.

Weiterhin ist bevorzugt, wenn ein Anteil von Fe an der Legierung 5.0 bis 12.0 Gew.%, insbesondere 7.5 bis 10.5 Gew.% beträgt.

Der Anteil von C an der Legierung beträgt vorzugsweise 0.03 bis 0.06 Gew.%.

Bevorzugt ist weiterhin, wenn ein Anteil von N an der Legierung 0.1 bis 0.5 Gew.%, insbesondere 0.1 bis 0.2 Gew.% beträgt.

Ferner ist bevorzugt, wenn ein Anteil von Cr an der Legierung 20.0 bis 28.0 Gew.%, insbesondere 21.0 bis 26.0 Gew.% beträgt. In fester Lösung befindliches Chrom steigert die Festigkeit. Chrom ist aber auch im Wesentlichen für die Korrosions- und Oxidationsbeständigkeit verantwortlich. Ein hoher Chromgehalt bedeutet eine hohe Korrosionsbeständigkeit. Die erfindungsgemäßen Legierungen besitzen damit einen hohen Widerstand gegen lokale Korrosion, den so genannten Lochfraß. Dieser Widerstand wird beschrieben durch die Wertzahl PRE (Pitting Resistance Equivalent). Der PRE liegt im Bereich von 34 - 89, bevorzugt 46 - 73, besonders bevorzugt 46 - 58.

Das PRE und damit die Beständigkeit gegen Lochfraß wird durch Schwefel um den Faktor 335 und Phosphor durch den Faktor 1.000 reduziert. Daher ist bevorzugt, wenn die kumulierte Verunreinigung mit P und S höchstens 0.03 Gew.% beträgt.

Ferner ist bevorzugt, wenn ein Anteil von W an der Legierung 14.0 bis 18.0 Gew.%, insbesondere 14.5 bis 15.5 Gew.% beträgt. Neben der Mischkristallhärtung und der Erhöhung der Reibbeständigkeit durch Wolfram, ist insbesondere im Anwendungsbereich als vaskuläre Gefäßstütze die erhöhte Werkstoffdichte durch Wolfram vorteilhaft, da dadurch eine gute Röntgensichtbarkeit erreicht wird. Im Weiteren trägt Wolfram zur Korrosionsbeständigkeit bei.

Ein Anteil von Si an der Legierung beträgt vorzugsweise 0.10 bis 0.25 Gew.%, insbesondere 0.12 bis 0.16 Gew.%.

Die Erfindung betrifft ferner die Verwendung der vorgenannten Kobaltlegierung zur Herstellung eines Stents.

Die Legierungen sind analog den üblichen Herstellverfahren für Kobaltbasis-Legierungen herstellbar.

Ausführungsbeispiel 1

Co-22Cr-15W-8Fe-8Mn-0.15Si-0.15N-0.05C

PRE = 51,25

Mechanische Eigenschaften im rekristallisierten Zustand nach einer Glühung bei ca. 1200°C:

$R_{p0,2}$ = 500 - 550 MPa
$R_m$ = 1000 - 1050 MPa
A > 60 %

Ausführungsbeispiel 2

Co-25Cr-10W-10Fe-8Mn-0.2Si-0.2N-0.05C

PRE = 46,0

Mechanische Eigenschaften im rekristallisierten Zustand nach einer Glühung bei ca. 1200°C:

$R_{p0,2}$ > 550 MPa

$R_m$ > 1100 MPa

A = 40 - 45 %

Ausführungsbeispiel 3

Co-25Cr-15W-10Fe-8Mn-0.2Si-0.15N-0.05C

PRE = 54,25

Mechanische Eigenschaften im rekristallisierten Zustand nach einer Glühung bei ca. 1200°C:

$R_{p0,2}$ > 550 MPa
$R_m$ > 1100 MPa
A = 40 - 45 %

**Patentansprüche**

1. Stent bestehend ganz oder in Teilen aus einer Kobaltlegierung der Zusammensetzung

Co:     18.36 - 66.85 Gew.%
Cr:     17.0 - 30.0 Gew.%
Mn:      4.0 - 10.0 Gew.%
W:      9.0 - 18.0 Gew.%
Fe:      3.0 - 20.0 Gew.%

C: 0.03 - 0.5 Gew.%
N: 0.1 - 1.0 Gew.%
Si: 0 - 2.0 Gew.%
O: 0 - 0.05 Gew.%

wobei sich die genannten Legierungskomponenten und herstellungsbedingte Verunreinigungen auf 100 Gew.% summieren und

(i) eine Wertzahl PRE für Korrosionsbeständigkeit, die sich aus den Gew.%-Anteilen der genannten Legierungskomponenten nach der Formel (1)

$$PRE = [Cr] + 1.65 \times [W] + 30 \times [N] \quad (1)$$

ergibt, im Bereich von 34 - 89 liegt; und
(ii) für den Gehalt an Stickstoff und Kohlenstoff folgende Einschränkungen nach den Formeln (2) und (3) gelten

$$0{,}15 \leq C + N \leq 1{,}00 \qquad (2)$$

$$0{,}25 \leq C/N \leq 1{,}00 \qquad (3).$$

2. Stent nach Anspruch 1, bei dem ein Anteil von Cr an der Legierung 20.0 bis 28.0 Gew.% beträgt.

3. Stent nach Anspruch 1, bei dem ein Anteil von Mn an der Legierung 6.0 bis 10.0 Gew.% beträgt.

4. Stent nach Anspruch 1, bei dem ein Anteil von W an der Legierung 14.0 bis 18.0 Gew.% beträgt.

5. Stent nach Anspruch 1, bei dem ein Anteil von Fe an der Legierung 5.0 bis 12.0 Gew.% beträgt.

6. Stent nach Anspruch 1, bei dem ein Anteil von Si an der Legierung 0.10 bis 0.25 Gew.% beträgt.

7. Stent nach Anspruch 1, bei dem ein Anteil von C an der Legierung 0.03 bis 0.06 Gew.% beträgt.

8. Stent nach Anspruch 1, bei dem ein Anteil von N an der Legierung 0.1 bis 0.5 Gew.% beträgt.

9. Stent nach Anspruch 1, bei dem die Wertzahl PRE im Bereich von 46 bis 73 liegt.

10. Stent nach Anspruch 1, bei dem eine kumulierte Verunreinigung mit P und S höchstens 0.03 Gew.% beträgt.

11. Verwendung einer Kobaltlegierung der Zusammensetzung

Co: 18.36 - 66.85 Gew.%
Cr: 17.0 - 30.0 Gew.%
Mn: 4.0 - 10.0 Gew.%
W: 9.0 - 18.0 Gew.%
Fe: 3.0 - 20.0 Gew.%
C: 0.03 - 0.5 Gew.%
N: 0.1 - 1.0 Gew.%
Si: 0 - 2.0 Gew.%
O: 0 - 0.05 Gew.%

wobei sich die genannten Legierungskomponenten und herstellungsbedingte Verunreinigungen auf 100 Gew.% summieren und

(i) eine Wertzahl PRE für Korrosionsbeständigkeit, die sich aus den Gew.%-Anteilen der genannten Legierungskomponenten nach der Formel (1)

$$PRE = [Cr] + 1.65 \text{ x } [W] + 30 \text{ x } [N] \quad (1)$$

ergibt, im Bereich von 34 - 89 liegt; und
(ii) für den Gehalt an Stickstoff und Kohlenstoff folgende Einschränkungen nach den Formeln (2) und (3) gelten

$$0{,}15 \leq C + N \leq 1{,}00 \qquad\qquad (2)$$

$$0{,}25 \leq C/N \leq 1{,}00 \qquad\qquad (3),$$

zur Herstellung eines Stents.

**Claims**

1. A stent made entirely or partially of a cobalt alloy having the following composition:

| | |
|---|---|
| Co: | 18.36-66.85 % by weight |
| Cr: | 17.0-30.0 % by weight |
| Mn: | 4.0-10.0 % by weight |
| W: | 9.0-18.0 % by weight |
| Fe: | 3.0-20.0 % by weight |
| C: | 0.03-0.5 % by weight |
| N: | 0.1-1.0 % by weight |
| Si: | 0-2.0 % by weight |
| O: | 0-0.05 % by weight |

wherein the alloy components listed above and production-related impurities add up to 100 % by weight and

(i) a PRE value for corrosion resistance, which is derived from the weight percentages of the aforementioned alloy components according to formula (1)

$$PRE=[Cr]+1.65\times[W]+30\times[N] \quad (1),$$

ranges between 34 and 89; and
(ii) for the contents of nitrogen and carbon the following restrictions according to formulas (2) and (3) apply

$$0.15 \leq C+N \leq 1.00 \qquad (2)$$

$$0.25 \leq C/N \leq 1.00 \qquad (3).$$

2. The stent according to Claim 1, wherein the proportion of Cr in the alloy is 20.0 to 28.0 % by weight.

3. The stent according to Claim 1, wherein a proportion of Mn in the alloy is 6.0 to 10.0 % by weight.

4. The stent according to Claim 1, wherein a proportion of W in the alloy is 14.0 to 18.0 % by weight.

5. The stent according to Claim 1, wherein a proportion of Fe in the alloy is 5.0 to 12.0 % by weight.

6. The stent according to Claim 1, wherein a proportion of Si in the alloy is 0.10 to 0.25 % by weight.

7. The stent according to Claim 1, wherein a proportion of C in the alloy is 0.03 to 0.06 % by weight.

8. The stent according to Claim 1, wherein a proportion of N in the alloy is 0.1 to 0.5 % by weight.

9. The stent according to Claim 1, wherein the PRE value ranges between 46 and 73.

10. The stent according to Claim 1, wherein a cumulative contamination with P and S is at most 0.03 % by weight.

11. Use of a cobalt alloy having the following composition:

Co: 18.36-66.85 % by weight
Cr: 17.0-30.0 % by weight
Mn: 4.0-10.0 % by weight
W: 9.0-18.0 % by weight
Fe: 3.0-20.0 % by weight
C: 0.03-0.5 % by weight
N: 0.1-1.0 % by weight
Si: 0-2.0 % by weight
O: 0-0.05 % by weight

wherein the alloy components listed above and production-related impurities add up to 100 % by weight and

(i) a PRE value for corrosion resistance, which is derived from the weight percentages of the aforementioned alloy components according to formula (1)

$$PRE=[Cr]+1.65\times[W]+30\times[N]\,(1),$$

ranges between 34 and 89; and
(ii) for the contents of nitrogen and carbon the following restrictions according to formulas (2) and (3) apply

$$0.15\leq C+N\leq1.00 \qquad (2)$$

$$0.25\leq C/N\leq1.00 \qquad (3),$$

for the production of a stent.

## Revendications

1. Endoprothèse constituée en totalité ou en partie par un alliage de cobalt de composition

Co : 18,36 - 66,85 % en poids
Cr: 17,0 - 30,0 % en poids
Mn : 4,0 - 10,0 % en poids
W : 9,0 - 18,0 % en poids
Fe : 3,0 - 20,0 % en poids

C : 0,03 - 0,5 % en poids
N : 0,1 - 1,0 % en poids
Si : 0 - 2,0 % en poids
O : 0 - 0,05 % en poids

où la somme des composants de l'alliage cités et des impuretés dues à la fabrication est de 100 % en poids, et où

(i) un nombre équivalent de résistance à la corrosion par piqûre PRE, pour la résistance à la corrosion, qui se définit, à partir des proportions en % en poids des composants de l'alliage cités, d'après la formule (1)

$$PRE = [Cr] + 1,65 \times [W] + 30 \times [N] \quad (1)$$

se situe dans un intervalle entre 34 et 89 ; et où
(ii) les restrictions suivantes pour les teneurs en azote et en carbone sont données par les formules (2) et (3)

$$0,15 \leq C + N \leq 1,00 \quad (2)$$

$$0,25 \leq C/N \leq 1,00 \quad (3)$$

2. Endoprothèse selon la revendication 1, dans laquelle la proportion de Cr dans l'alliage se situe de 20,0 à 28,0 % en poids.

3. Endoprothèse selon la revendication 1, dans laquelle la proportion de Mn dans l'alliage se situe de 6,0 à 10,0 % en poids.

4. Endoprothèse selon la revendication 1, dans laquelle la proportion de W dans l'alliage se situe de 14,0 à 18,0 % en poids.

5. Endoprothèse selon la revendication 1, dans laquelle la proportion de Fe dans l'alliage se situe de 5,0 à 12,0 % en poids.

6. Endoprothèse selon la revendication 1, dans laquelle la proportion de Si dans l'alliage se situe de 0,10 à 0,25 % en poids.

7. Endoprothèse selon la revendication 1, dans laquelle la proportion de C dans l'alliage se situe de 0,03 à 0,06 % en poids.

8. Endoprothèse selon la revendication 1, dans laquelle la proportion de N dans l'alliage se situe de 0,1 à 0,5 % en poids.

9. Endoprothèse selon la revendication 1, dans laquelle le nombre PRE se situe dans l'intervalle de 46 à 73.

10. Endoprothèse selon la revendication 1, dans laquelle une impureté cumulée contenant du P et du S atteint au maximum 0,03 % en poids.

11. Utilisation d'un alliage de cobalt de composition

Co:     18,36 - 66,85 % en poids
Cr:     17,0 - 30,0 % en poids
Mn:     4,0 - 10,0 % en poids
W:      9,0 - 18,0 % en poids
Fe:     3,0 - 20,0 % en poids
C:      0,03 - 0,5 % en poids

N:        0,1 - 1,0 % en poids
Si:       0 - 2,0 % en poids
O:       0 - 0,05 % en poids

où la somme des composants de l'alliage cités et des impuretés dues à la fabrication est de 100 % en poids, et où

(i) un nombre équivalent de résistance à la corrosion par piqûre PRE, pour la résistance à la corrosion, qui se définit, à partir des proportions en % en poids des composants de l'alliage cités, d'après la formule (1)

$$PRE = [Cr] + 1{,}65 \text{ x } [W] + 30 \text{ x } [N] \quad (1)$$

se situe dans un intervalle entre 34 et 89 ; et où

(ii) les restrictions suivantes pour les teneurs en azote et en carbone sont données par les formules (2) et (3)

$$0{,}15 \le C + N \le 1{,}00 \quad (2)$$

$$0{,}25 \le C/N \le 1{,}00 \quad (3)$$

pour la fabrication d'une endoprothèse.

**IN DER BESCHREIBUNG AUFGEFÜHRTE DOKUMENTE**

**In der Beschreibung aufgeführte Patentdokumente**

- DE 19704530 A1
- US 3865585 A
- DE 3624377 A1
- WO 2011118615 A
- EP 2551363 A
- US 2010114304 A